(19) Europäisches Patentamt
European Patent Office
Office européen des brevets

(11) **EP 3 491 367 B1**

(12) **EUROPÄISCHE PATENTSCHRIFT**

(45) Veröffentlichungstag und Bekanntmachung des Hinweises auf die Patenterteilung:
**05.08.2020   Patentblatt 2020/32**

(51) Int Cl.:
*G01N 23/046* (2018.01)    *A61B 6/00* (2006.01)
*A61B 6/03* (2006.01)

(21) Anmeldenummer: **17749165.1**

(22) Anmeldetag: **28.07.2017**

(86) Internationale Anmeldenummer:
**PCT/EP2017/069193**

(87) Internationale Veröffentlichungsnummer:
**WO 2018/020009 (01.02.2018 Gazette 2018/05)**

(54) **VERFAHREN UND VORRICHTUNG ZUM KALIBRIEREN EINES RÖNTGENSYSTEMS**

METHOD AND DEVICE FOR CALIBRATING AN X-RAY SYSTEM

PROCÉDÉ ET DISPOSITIF D'ÉTALONNAGE D'UN SYSTÈME RADIOGRAPHIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2016   DE 102016214062**

(43) Veröffentlichungstag der Anmeldung:
**05.06.2019   Patentblatt 2019/23**

(73) Patentinhaber: **Fraunhofer-Gesellschaft zur Förderung der angewandten Forschung e.V.
80686 München (DE)**

(72) Erfinder:
• **AMR, Mahmoud
  90768 Fürth (DE)**
• **SCHÖN, Tobias
  90453 Nürnberg (DE)**

(74) Vertreter: **Pfitzner, Hannes et al
Schoppe, Zimmermann, Stöckeler
Zinkler, Schenk & Partner mbB
Patentanwälte
Radlkoferstraße 2
81373 München (DE)**

(56) Entgegenhaltungen:
WO-A1-2012/007036      WO-A1-2015/051468
US-A1- 2012 201 352

**Beschreibung**

Ausführungsbeispiele der vorliegenden Erfindung beziehen sich auf ein Verfahren und eine Vorrichtung zur Röntgen-basierten Kalibrierung und Justage von Achssystemen, z.B. Achssysteme eines Röntgensystems bzw. eines Computertomographiesystems.

In der Computertomographie müssen für die Aufnahme und Rekonstruktion von Daten ein justiertes Achssystem sowie Wissen über die Lage von Brennfleck und Abbildungsebene relativ zum Achssystem vorhanden sein. Insbesondere bei robotergestützten CT Anlagen stellt dies eine hohe Herausforderung an die Präzision bei der Fertigung und beim Aufbau des Systems dar. Daher ist es oft notwendig, die Größen nach Aufbau des Systems mit Hilfe von Algorithmen zur Kalibrierung oder Korrektur zu bestimmen. Siehe z.B. WO 2015/051468.

Hier spielen insbesondere die Kenntnisse über die Konfiguration des Achssystems eine Rolle. Diese werden bei Laboranlagen für Kreis- oder HelixCT häufig aufwändig von Hand justiert, bis eine manuell überprüfte Bildqualität in 2D und 3D erreicht wird. Der Austausch von Komponenten wie z. B. Röntgenröhre oder Detektor erfordert dabei oft eine erneute Justage des Systems. Komplexere Anlagen mit aufwändigeren Trajektorien können mit klassischen Ansätzen nicht kalibriert werden, da die Bahn von zu vielen Freiheitsgraden abhängt oder die Manipulatoren frei im Raum positioniert werden.

Aktuelle Verfahren basieren in der Regel auf fest definierten Trajektorien (wie Kreis, Helix), sehr präzisen Referenzkörpern und/oder bestimmen nur eingeschränkte Freiheitsgrade des Systems (wie Detektor Misalignment in zwei Dimensionen). Dabei werden oft Annahmen über die Genauigkeit des Achssystems getroffen (z. B. exakte Rotationsachse). Algorithmen aus der Robotik basieren zumeist entweder auf taktilem Zugang oder der Vermessung mittels Laser. Beide Varianten können für CT Komponenten nicht angewendet werden. Bisherige Ansätze für CT-Systeme lassen sich in verschiedene Grundsätze untergliedern.

- Verfahren zur Berechnung der Lage von Quelle und Detektor, relativ zum Objekt und unabhängig vom Achssystem
- Verfahren zur Berechnung der Basen und Werkzeugkoordinatensysteme von Industrierobotern

Verfahren zur Kalibrierung von Industrierobotern basieren in der Regel auf die freie Zugänglichkeit des Werkzeugs oder die Möglichkeit, dieses mittels optischer Verfahren beobachten zu können. Dies ist bei den in der CT ein- gesetzten Komponenten Röntgenröhre und Detektor nicht möglich, da hier der Brennfleck der Röntgenröhre sowie die Abbildungsebene des Detektors als Werkzeug betrachtet wird und diese Punkte nicht zugänglich sind. Hier wird im Weiteren lediglich auf Verfahren zur Bestimmung der Lage der Basen der Manipulatoren eingegangen.

UltraCal ist ein optisches Kalibrierverfahren zur Bestimmung der Basen zweier Roboter, welches auf einem Laser mit integriertem Sensor und einem Retroreflektor basiert.

Laser und Retroreflektor werden an jeweils einem Roboter befestigt und von Hand so ausgerichtet, dass die Abweichung der Laserreflektion in zwei Raumdimensionen nahezu Null beträgt. Durch Manipulation der Roboterstellung wird nun die relative Lage der Roboter zueinander bestimmt. Dies muss für mindestens drei Punkte im Raum durchgeführt werden
Ähnlich zu UltraCal wird ein z. B. durch eine Kugel definierter Raumpunkt von den Robotern abgetastet, oder diese berühren sich mit Hilfe spezieller Halterungen. Die für die Rekonstruktion wichtigen Größen wie Lage des Brennflecks und der Abbildungsebene können so nicht bestimmt werden.

Es existieren sehr viele Verfahren zur Bestimmung der sog. Misalignments von Quelle und Detektor. Hier werden über unterschiedliche Ansätze die Lage von Quelle und Detektor zum Objektkoordinatensystem bestimmt. Nahezu jedes Verfahren löst entweder nur eine sehr beschränkte Menge von Freiheits- graden oder hat starke Einschränkungen, z. B. auf die gefahrene Trajektorie (nur Kreis, nur Helix, ...). Andere Verfahren wiederum benötigen eine fest vorgegebene Anordnung von Kugeln in einem Objekt oder eine enorm hohe Genauigkeit bei der Herstellung des Körpers. Diese Verfahren sind des Weiteren nicht dazu geeignet, die eigentlichen Manipulatoren der Komponenten zu kalibrieren. Deshalb besteht der Bedarf nach verbessertem Ansatz.

Aufgabe der vorliegenden Erfindung ist es ein Verfahren zur Kalibrierung eines Röntgensystems, insbesondere eines Computertomographiesystems mit sowohl unabhängig voneinander beweglichen Strahlenquellen und Strahlendetektor zu schaffen, das hinsichtlich Ergonomie und Kalibrierungsqualität verbessert ist.

Die Aufgabe wird durch die unabhängigen Patentansprüche gelöst.

Ausführungsbeispiele der vorliegenden Erfindung schaffen ein Verfahren zum Kalibrieren mit den Schritten Ermitteln eines kinematischen Modells des Röntgensystems mit allen kinematischen Beziehungen für zumindest eine erste Position auf der Bewegungstrajektorie und Beschreiben derselben anhand eines ersten Kinematikparametersatzes und anhand von zu kalibrierenden Parametern. Hierbei definiert der erste Kinematikparametersatz zusammen mit den zu kalibrierenden Parametern eine erste Gleichung eines Gleichungssystems. Bei dem Kinematikmodell können sowohl die zwei Roboter mit dem Strahlendetektor bzw. der Strahlenquelle und beispielsweise auch ein Kalibrierkörper in dem kinematischen Modell inkludiert sein. Das kinematische Modell wird durch Kinematikparameter beschrieben. Diese können allgemein mit Mitteln der linearen Algebra wie z.B. Koordinatentransformationen sowie auch in komplexeren Formen z.B. mit (partiellen) Differentialgleichungen formuliert werden. Alternativ können diese mit Minimaldarstellungen wie beispielsweise Denavit-Hartenberg oder Hayati-Roberts beschrieben werden.

In einem Schritt nach dem Ermitteln der kinematischen Modelle werden die Startwerte für die zu kalibrierenden Parameter festgelegt und ein Kalibriervorgang begonnen. Der Kalibriervorgang umfasst das Ermitteln einer Röntgenprojektion des oben erwähnten Kalibrierkörpers für die erste Position auf der Bewegungstrajektorie, um für zumindest ein Merkmal des Kalibrierkörpers ein erstes Messergebnis zu erhalten. Durch Vergleich des ersten Messergebnisses mit der jeweiligen Referenz kann ein Fehlermaß bestimmt werden. Das oben erwähnte Gleichungssystem kann z.B. durch einfache Nullstellensuche oder dadurch gelöst werden, dass das Fehlermaß minimiert wird (z. B. anhand des Levenberg-Marquard-Algorithmus). Hierbei werden beispielsweise die zu kalibrierenden Parameter variiert bis das Fehlermaß ein Minimum erreicht hat, während die nicht veränderlichen Kinematikparameter des kinematischen Modells, wie z. B. Längen eines Schenkels eines Roboters, oder veränderliche Kinematikparameter, wie z. B. die Maschinenparameter, die eine Gelenkposition für den jeweiligen Freiheitgrad definieren, kann nicht variiert werden.

Der Kern der vorliegenden Erfindung basiert somit darauf, dass zur Kalibrierung des Systems ein vollständiges kinematisches Modell des Achssystems ausschließlich aufgrund von gemessenen Röntgen-Projektionen erstellt wird (z. B. Denavit-Hartenberg). Zur Bestimmung der zu lösenden kinematischen Parameter werden Röntgenprojektionen eines Referenzkörpers zumindest in einer, bevorzugt aber in unterschiedlichsten Achspositionen aufgenommen. Die Anzahl der Projektionen und Achspositionen variiert dabei je nach Anzahl der zu lösenden Freiheitsgrade. Das Fehlermaß für das zu lösende Gleichungssystem kann auf unterschiedliche Weise festgelegt und anschließend minimiert werden. Für ein Fehlermaß können zum einen die erzeugten Abbildungen als Referenzwerte dienen. Bei einem Referenzkörper aus Kugeln könnte z. B. der Tensor ersten oder zweiten Grades der Kugelabbildung als Referenz verwendet werden. Ein alternativer vielsprechender Ansatz wäre es, dies direkt im Objekt- anstatt Projektionsraum zu lösen, z. B. das Fehlermaß vergleicht die rekonstruierten Kugeln mit dem CAD-Modell. Dadurch würde man eine höhere Genauigkeit erwarten und zusätzliche Aspekte aus der realen Anlage könnten betrachtet werden, wie z. B. die Modellierung des Brennflecks (aktuell Lochkameramodell). Das Gleichungssystem kann durch eine geeignete Suchstrategie (z. B. nichtlineare Optimierungsverfahren) auf Basis des Modells gelöst werden. D. h. auf Basis des Modells und geeigneten Startwerten werden die gesuchten Parameter so bestimmt, dass die mit Hilfe des Modells erzeugten Abbildungen bzw. Rekonstruktionen den Referenzdaten entsprechen.

Vorteilhaft bei diesem vorgestellten Ansatz ist, dass zur Kalibrierung des Systems ein vollständiges kinematisches Modell des Achssystems ausschließlich aufgrund von gemessenen Röntgen-Projektionen erstellt wird (z. B. Denavit-Hartenberg) bzw. werden kann. Zur Bestimmung der zu lösenden kinematischen Parameter werden Röntgenprojektionen eines Referenzkörpers in unterschiedlichsten Achspositionen aufgenommen. Die Anzahl der Projektionen und Achspositionen variiert dabei je nach Anzahl der zu lösenden Freiheitsgrade. Das Fehlermaß für das zu lösende Gleichungssystem kann auf unterschiedliche Weise festgelegt und anschließend minimiert werden. Für ein Fehlermaß können zum einen die erzeugten Abbildungen als Referenzwerte dienen.

Entsprechend weiteren Ausführungsbeispielen können die Schritte "Ermitteln eines kinematischen Modells" für eine zweite Position und "Beschreiben desselben anhand eines zweiten Kinematikparametersatzes", "Ermitteln einer Röntgenprojektion des Kalibrierkörpers" für eine zweite Position (um ein zweites Messergebnis zu erhalten) und "Vergleich des (zweiten) Messergebnisses mit der jeweiligen Referenz" (um das Fehlermaß erneut zu bestimmen) wiederholt werden. Der zweite Kinematikparametersatzes bildet zusammen mit den zu kalibrierenden Parametern eine zweite Gleichung des Gleichungssystems, so dass dieser Ansatz ermöglicht, mehrere unbekannte (zu kalibrierenden) Parameter zu finden. Hier wird die Annahme getroffen, dass die zu kalibrierenden Parameter über die Kinematikparametersätze konstant sind.

Das Gleichungssystem kann durch eine geeignete Suchstrategie (z. B. nichtlineare Optimierungsverfahren) auf Basis des Modells gelöst werden. D. h. auf Basis des Modells und geeigneten Startwerten werden die gesuchten Parameter so bestimmt, dass die mit Hilfe des Modells erzeugten Abbildungen bzw. Rekonstruktionen den Referenzdaten entsprechen. Der Referenzkörper kann dabei Teil des Modells sein, so dass dessen Geometrie bei Bedarf mitbestimmt werden kann.

Dieser Ansatz ermöglicht, ein beliebiges Achssystem/Manipulationssystem auf Basis von Röntgenprojektionen eines Referenzkörpers zu kalibrieren. In diesem Kontext beschreibt Kalibrieren insbesondere die Bestimmung der dem Achssystem und seinen Konfiguration zugrundeliegenden Koordinatentransformationen, mit der Zielsetzung ein vollständiges kinematisches Modell des Achssystems aufgrund von gemessenen Röntgenprojektionen zu erstellen.

Auch wenn das Konzept auf jegliche Achssysteme (z.B. eines Helix- oder Rotations-CTs) anwendbar ist, können ausgehend von CT-Systemen mit einem oder mehreren, wie z.B. zwei (mehrgliedrigen) Robotern, die z.B. einerseits die Strahlenquelle und andererseits den Strahlendetektor bewegen, die zu kalibrierenden Parameter beispielsweise der Versatz zwischen den zwei Robotern sein, der noch genauer bestimmt werden muss. Weitere zu kalibrierende Parameter können der Versatz zwischen Strahlenquelle und dem bekannten kinematischen Modell des Roboters oder der Versatz zwischen dem Röntgendetektor und dem kinematischen Modell des Roboters sein.

In einer ersten Näherung werden diese Kinematikparameter beispielsweise gemessen oder aus einem bekannten Datensatz, z. B. CAD-Daten, abgelesen. Im Regelfall reicht dieses messtechnisch ermittelte Ergebnis allerdings nicht dazu aus, um CT-Rekonstruktionen zu ermöglichen.

Entsprechend einem Ausführungsbeispiel kann das Gleichungssystem beispielsweise wie folgt gelöst werden:

$$\min_{\vec{u},\vec{p}} \left\| \frac{1}{NK} \sum_{n=1}^{N} \sum_{k=1}^{K} e_{n,k} \right\|_2 ,$$

wobei

$$e_{n,k}(\vec{u}, \vec{q_n}, \vec{p_k}) = \| f - f_{ref} \|_2 ;$$

und

wobei *u* die zu kalibrierenden Parameter beschreibt und $f(\vec{u}, \vec{q}, \vec{p}) \rightarrow \vec{p}_e$, wenn nur ein Merkmal des Kalibrierobjekts verwendet wird, oder $f(\vec{u}, \vec{q_u}, \vec{p_k}) \rightarrow \vec{p_k}'$, wenn mehrere Merkmale des Kalibrierkörpers durch mehrere Projektionen ermittelt werden. Hier beschreibt q die jeweiligen Maschinenkoordinaten, d.h. die Gelenkstellungen des Achssystems, beschrieben anhand der Koordinaten des kinematischen Modells, und wobei p bzw. $p_k$ die Position des Merkmals bzw. der Merkmale des Kalibrierkörpers für das erste und/oder das zweite Messergebnis mit dem Koordinatensystem des kinematischen Modells beschreibt. P' bzw. $p_k$' beschreibt die Position der Abbildung des Merkmals / der Merkmale auf den Detektor mit den Koordinaten des kinematischen Modells.

Entsprechend Ausführungsbeispielen werden die jeweiligen Referenzen durch den Tensor des ersten oder des zweiten Grades gebildet. Diese Variante eignet sich insbesondere bei Kalibrierobjekten aus Kugeln. Hierbei kann - entsprechend weiteren Ausführungsbeispielen - auf folgende Formel zugegriffen werden:

$$M_{pq} = \int_{-\infty}^{\infty} \int_{-\infty}^{\infty} x^p y^q f(x,y)\, dx\, dy .$$

Alternativ hierzu kann die jeweilige Referenz auch rechentechnisch, z. B. bei Kenntnis der Position des Kalibrierkörpers und der Geometrie des Kalibrierkörpers ermittelt werden.

An dieser Stelle sei angemerkt, dass entsprechend Ausführungsbeispielen das Positionsmodell des jeweiligen Strahlenquellen-Detektor-Paares mit berücksichtigt wird. Dies geschieht beispielsweise anhand folgender Formel:

$$\begin{bmatrix} v_x & w_x & d_x - s_x \\ v_y & w_y & d_y - s_y \\ v_z & w_z & d_z - s_z \end{bmatrix} \begin{bmatrix} \lambda x' \\ \lambda y' \\ \lambda \end{bmatrix} = \begin{bmatrix} p_x - s_x \\ p_y - s_y \\ p_z - s_z \end{bmatrix},$$

wobei $p_x$, $p_y$ und $p_z$ die Position des Merkmals des Kalibrierkörpers im Raum beschreibt, wobei $s_x$, $s_y$ und $s_z$ die Position des Projektionszentrums im Raum beschreibt, wobei $d_x$, $d_y$ und $d_z$ die Position der Abbildungsebene im Raum beschreibt, wobei $v_x$, $v_y$ und $v_z$ sowie $\omega_x$, $\omega_y$ und $\omega_z$ die ebenen Basisvektoren sind. S, d, v und ω sind mit den Koordinaten des kinematischen Modells beschrieben.

Ein weiteres Ausführungsbeispiel bezieht sich auf eine entsprechende Vorrichtung, die das oben beschriebene Verfahren ausführt. Hierbei ist eine Berechnungseinheit zur Ermittlung der kinematischen Modelle sowie ein Kalibrierer zum Durchführen des Kalibrierverfahrens vorgesehen.

Ein weiteres Ausführungsbeispiel bezieht sich auf ein Computerprogramm zur Durchführung eines der oben beschriebenen Verfahren. Hierdurch kann eine Automatisierbarkeit des Verfahrens, d. h. ohne Benutzerinteraktion, ermöglicht werden.

Ein weiteres Ausführungsbeispiel bezieht sich auf ein Röntgensystem mit beispielsweise zwei Robotern, die Strahlenquelle und Röntgenquelle bewegen können, und der oben erläuterten Vorrichtung bzw. einem Computer zur Durchführung eines der oben beschriebenen Verfahren. Hierbei kann entsprechend weiteren Ausführungsbeispielen die oben erläuterte Vorrichtung bzw. der Computer auch eine Schnittstelle zum Steuern des Röntgensystems oder eine Steuerung zum Steuern der Roboter des Röntgensystems umfassen.

Weiterbildungen sind in den Unteransprüchen definiert. Ausführungsbeispiele werden anhand der beiliegenden Figuren erläutert. Es zeigen:

Fig. 1a  ein schematisches Flussdiagramm des Verfahrens beim Kalibrieren eines Röntgensystems mit beweglicher Strahlenquelle und beweglichen Strahlendetektor gemäß einem Ausführungsbeispiel;

Fig. 1b  ein schematisches Blockschaltbild von zwei mehrgliedrigen Robotern zur Bewegung von Strahlenquelle und Strahlendetektor gemäß einem Ausführungsbeispiel;

Fig. 1c  ein schematisches Flussdiagramm eines erweiterten Verfahrens beim Kalibrieren eines Röntgensystems mit beweglicher Strahlenquelle und beweglichen Strahlendetektor gemäß einem weiteren Ausführungsbeispiel; und

Fig. 2a,b Tabellen zur Illustration von kinematischen Parametern bzw. kinematischen Variablen.

Bevor nachfolgend Ausführungsbeispiele der vorliegenden Erfindung anhand der beiliegenden Zeichnungen erläutert werden, sei darauf hingewiesen, dass gleichwirkende Elemente und Strukturen mit gleichen Bezugszeichen versehen sind, so dass die Beschreibung derer aufeinander anwendbar bzw. austauschbar ist.

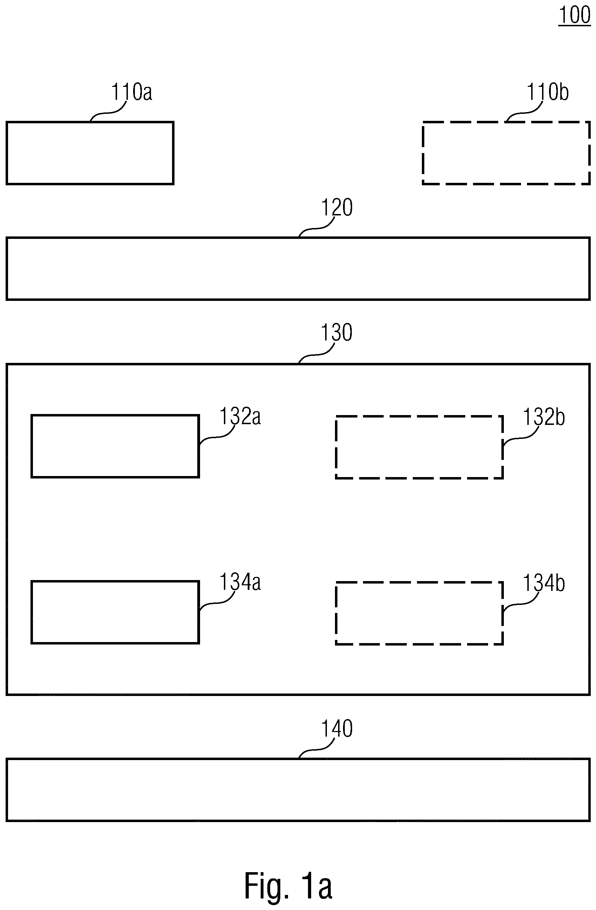

Fig. 1a zeigt die Basisschritte eines Verfahrens 100 beim Kalibrieren. Das Verfahren 100 umfasst den Basisschritt 110a, den optionalen Schritt 110b sowie die Basisschritte 120, 130 und 140, wobei der Schritt 130 eine Vielzahl von Unterschritten umfasst.

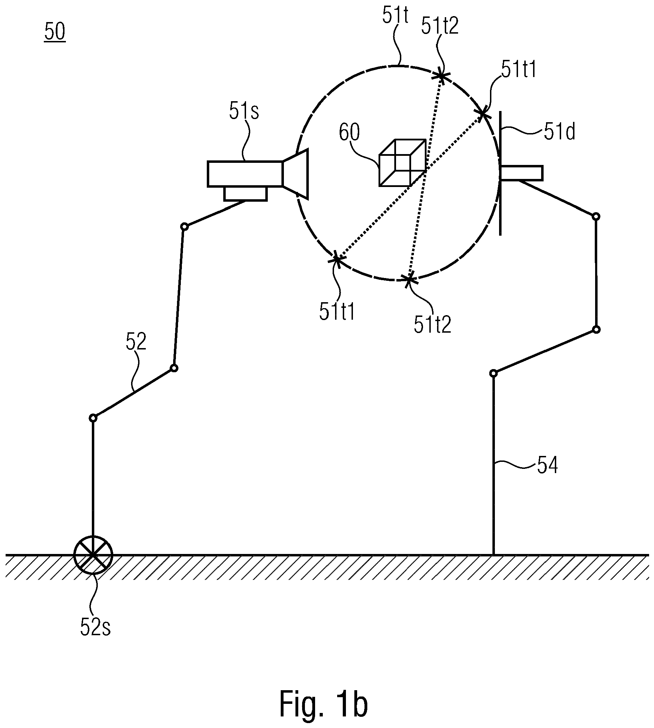

Die Schritte 110a und 110b sind im Wesentlichen die gleichen, wobei der obligatorische Schritt 110a für eine erste Position auf einer Bewegungsbahn des Röntgensystems (vgl. System 50 in Fig. 1b) und der optionale Schritt 110b für eine zweite Position des Röntgensystems ausgeführt wird. Hierbei sei kurz auf Fig. 1b verwiesen.

Fig. 1b zeigt zwei Roboter 52 und 54, hier mehrgliedrige Roboter, wobei der Roboter 52 die Strahlenquelle 51s und der Roboter 54 den Röntgendetektor 51d hält. Strahlenquelle 51s und Detektor 51d sind beispielsweise entlang der gepunkteten Bewegungsbahn 51t (Trajektorie) des Röntgensystems 50 bewegbar. Auf dieser Trajektorie gibt es eine Vielzahl von Positionen, wobei eben der Schritt 110a für eine Position 51t1 (vgl. Kreuze) und der Schritt 110b für eine Position 51t2 durchgeführt wird. Wie zu erkennen ist, gibt es jeweils zwei Kreuze 51t1 und 51t2, da zu einer Position auf der Trajektorie jeweils die zwei Positionen für Strahlendetektor 51d und Strahlenquelle 51s gehören. Die zwei Position sind nicht zwingend in einer Ebene, sondern können allein in jedem beliebigen Winkel im Raum um das Objekt 60 bzw. das Prüfobjekt angeordnet sein.

An diesen zwei Positionen 51t2 und 51t1 werden eben die Schritte 110a und 110b des Ermittelns eines kinematischen Modells den Röntgensystems 50 mit allen kinematischen Beziehungen für die erste und zweite Position 51t1 und 51t2 auf der Bewegungstrajektorie 51t durchgeführt. Im Schritt 110a und 110b werden Kinematikparameter eines Kinematikparametersatzes ermittelt, die das kinematische Modell des Gesamtsystems, umfassend die Roboter 52 und 54 zur Bewegung der Strahlenquelle 51s und des Strahlendetektors 51d, beschreiben.

Der erste Kinematikparametersatz, der in dem Schritt 110a ermittelt wird und der zweite Kinematikparameterdatensatz, der in dem Schritt 110b ermittelt wird, können beispielsweise mittels der Nomenklatur nach Denavit-Hartenberg erfasst sein. Die Denavit-Hartenberg-Konvention ermöglicht die Überführung von Ortskoordinatensystemen innerhalb von kinematischen Ketten. Somit ist es möglich, einen Roboter, insbesondere einen mehrgliedrigeren Roboter, wie er in Fig. 1b gezeigt ist, anhand von Denavit-Hartenberg-Parametern bzw. anhand von Homogenmatrizen derart zu beschreiben, dass klar wird, wie dieser aufgebaut zweigliedrig, dreigliedrig, ...) und dimensioniert (kurze Schenkel, lange Schenkel) ist und auch wie dessen Position zu einem Fixpunkt ist. Nach der Denavit-Hartenberg-Konvention sind folgende Voraussetzungen notwendig:

1. Die $z_{n-1}$ Achse liegt entlang der Bewegungsachse des n-ten Gelenks.
2. Die $x_n$ Achse ist das Kreuzprodukt von $z_{n-1}$ Achse und $z_n$ Achse.
3. Das Koordinatensystem wird durch yn so ergänzt, dass es ein rechtshändiges System gibt. In Folge dessen wird für das erste Gelenk die x Achse vom zweiten Gelenk übernommen.

Entsprechend der Denavit-Hartenberg-Konvention gibt es unterschiedliche Parameter, nämlich (1.) nicht veränderliche Kinematikparameter, wie z. B. Schenkellängen der Roboter 52 und 54, (2.) veränderliche Kinematikparameter, wie die Maschinenparameter, die die Bewegung oder Position des jeweiligen Gelenks der mehrgliedrigeren Roboter 52 und 54 beschreiben, sowie (3.) zu kalibrierende Parameter umfassen. Die zu kalibrierenden Parameter sind eben die Elemente, bezüglich welcher der Wert nicht oder nicht exakt bekannt ist. Diese können sich beispielsweise auf die Relativposition der zwei Roboter 52 und 54 zueinander beziehen oder die Länge eines Schenkels bei der Unsicherheit über die Korrektheit der angenommenen Werte besteht.

An dieser Stelle sei angemerkt, dass bevorzugterweise beide Roboter 52 und 54 in einem gemeinsamen kinematischen Modell entsprechend der Denavit-Hartenberg-Konvention beschrieben sind, wobei beispielsweise der Roboter 54 in dem Modell des Roboters 52, d. h. um den Ursprung bzw. Standort um den Roboter 52 (vgl. Punkt 52s) beschrieben wird. Dieser Roboter 54 hat gegenüber dem Roboter 52 einen Versatz, nämlich den Abstand dieser zwei Roboter, was im Regelfall eine zu kalibrierende Parameter ist, da hier die Positionierung variieren kann. Infolgedessen muss diese zu kalibrierende Parameter, d. h. also der Abstand zwischen den zwei Robotern 52 und 54 im Regelfall mit dem Verfahren 100 mitkalibriert werden.

Da der sowohl der erste Kinematikparametersatz aus dem Schritt 110a und der zweite Kinematikparametersatz aus dem Schritt 110b sich auf dasselbe Röntgensystem 50 und damit auch auf dieselbe Roboteranordnung der Roboter 52 und 54 beziehen, unterschieden sich diese zwei Datensätze nur im Hinblick auf die Maschinenparameter, da eben die Position 51t2 von der Position 51t1 durch Veränderung der Maschinenparameter erreicht wurde. Anders ausgedrückt heißt das, dass die nicht veränderlichen Kinematikparameter, wie z. b. die Achslänge und die zu kalibrierenden Parameter, wie z. B. der Abstand der Roboter 52 und 54 in beiden Kinematikparameterdatensätzen konstant sind.

Diese Eigenschaft wird sich bei dem Schritt 130 des Kalibrierens zunutze gemacht, wobei vor dem Kalibrieren erst einmal Startwerte für die zu kalibrierenden Parameter festgelegt werden müssen, um den Kalibriervorgang durchzuführen. Diese zu kalibrierenden Parameter werden mit Werten in dem Schritt 120 versehen. Hierbei können beispielsweise die Werte aus Messungen wie z. B. mittels Lasermessgeräten oder aus Konstruktionsdaten für das Röntgensystem 50 entstammen.

Der Schritt des Kalibrierens 130 umfasst zumindest zwei in der hier vorgestellten Variante vier Unterschritte 132a /132b und 134a /134b, wobei die Unterschritte 134a /134b für die zweite Position (wie oben) optional sind .

Der Schritt 132 sowohl a als auch b bezieht sich auf das Ermitteln einer Röntgenprojektion eines Kalibrierkörpers 60 (vgl. Fig. 1b), wobei wiederum der Schritt a für die Position 51t1 und der Schritt b für die Position 51t2 durchgeführt wird. Das Ergebnis der Schritte 132a und b sind jeweils Messungen, d. h. z. B. eine Abbildung eines Schwerpunktes (als Merkmal) des Kalibrierkörpers 60 auf den Röntgendetektor 51d, und zwar um genau zu sein, auf eine Position des Röntgendetektors 51d. An dieser Stelle sei angemerkt, dass die Position des Kalibrierkörpers 60 oder insbesondere die Position des Merkmals des Kalibrierkörpers 60, anhand dessen kalibriert wird (bzw. der Merkmale, anhand derer kalibriert wird) auch in dem kinematischen Modell mitinkludiert sind, d. h. anhand des Koordinatensystems das kinematische Modells beschrieben werden.

Bei dem Kalibrierkörper 60 bzw. Referenzkörper handelt es sich beispielsweise um einen derartigen, bei dem hinreichende Informationen bezüglich der erhaltenen Strukturen bzw. der Merkmale/Features vorliegen. Diese Merkmale können beispielsweise aus einem CAD-Modell entstammen. Ein solches Objekt könnte z. B. aus einer beliebigen Anordnung von Kugeln bestehen. An den Referenzkörper 60 werden keine speziellen Anforderungen bezüglich der Genauigkeit oder der Anordnung der Kugeln gestellt. Der Referenzkörper kann dabei Teil des Modells sein, so dass dessen Geometrie bei Bedarf mitbestimmt werden kann. Im Allgemeinen kann sogar das zu messende Objekt als Referenzkörper verwendet werden.

Bei einem Referenzkörper 60 aus Kugeln könnte z. B. der Tensor ersten oder zweiten Grades der Kugelabbildung als Referenz verwendet werden. Ein alternativer vielversprechender Ansatz wäre es, dies direkt im Objekt, anstatt Projektionsraum, zu lösen, z. B. das Fehlermaß vergleicht die rekonstruierten Kugeln mit dem CAD-Modell. Dadurch würde man eine höhere Genauigkeit erwarten und zusätzliche Aspekte aus der realen Anlage könnten betrachtet werden, wie z. B. die Modellierung des Brennflecks (aktuell Lochkameramodell).

Wenn man nun ausgehend von der nun bekannten Projektionsposition bei der Kalibrierung kann diese mit einer erwarteten Referenz, d.h. also beispielsweise mit einer rechentechnisch ermittelten Projektionsposition oder mit einer aus der Messung selbst abgeleiteten Projektionsreferenz verglichen werden, um ein sogenanntes Fehlermaß zu bestimmen. Dieser Schritt wird sowohl für die Messung des Schritts 132a als auch für die Messung des Schrittes 132b in den Schritten 134a und 134b durchgeführt.

Wenn man nun in einem nachgelagerten Schritt eben dieses Fehlermaß minimiert, indem beispielsweise die zu kalibrierenden Parameter entsprechend angepasst werden, kommt man zu den gesuchten Kalibrierdaten. Dieser Schritt entspricht dem zentralen Schritt 140, da das Minimieren durch Lösen eines Gleichungssystems durchgeführt wird. Beim Gleichungssystem handelt es sich um eines, das je Position 51t1 und 51t2 eine Kinematikgleichung umfasst, d. h. also um ein Gleichungssystem mit mehreren Gleichungen und mehreren Unbekannten bzw. Variablen, so dass dieses dadurch lösbar wird, dass beliebig viele neue Gleichungen durch erneute Kalibriervorgänge an weiteren Positionen hinzugefügt werden können. Alternativ kann das Gleichungssystem auch durch einfache Nullstellensuche gelöst werden.

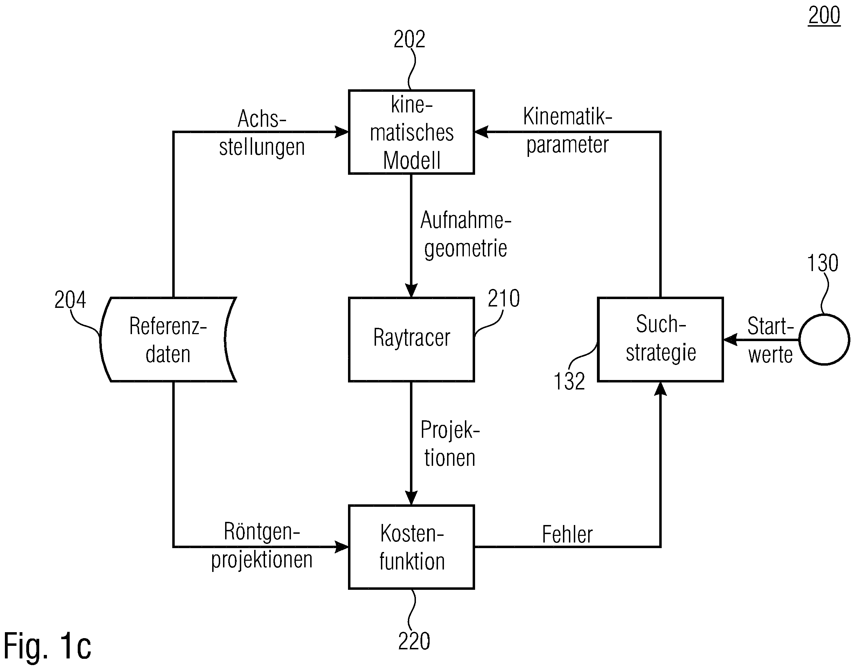

Wie anhand von Fig. 1c dargestellt ist, kann das Verfahren auch etwas komplexer ausgeführt sein. Fig. 1c zeigt das Verfahren 200 mit den Schritten Startwerte festlegen 130, Definieren einer Suchstrategie 132 ausgehend von den festgelegten Kinematikparametern im kinematischen Modell 202.

Das kinematische Modell 202 umfasst neben den Kinematikparametern auch die Achsstellungen, d. h. also die Maschinenkommandos, die den Referenzdaten 204 entstammen. Ausgehend von dem nun festgelegten kinematischen Modell 202 werden die Aufnahmegeometrien abgeleitet und die entsprechenden Projektionen (vgl. Schritt Raytracer 210) genommen. Aus den Referenzdaten stammen auch der Erwartungshorizont für die Röntgenprojektionen, die dann mittels einer Kostenfunktion (vgl. Schritt 220) verglichen werden. Hieraus wird der Fehler abgeleitet und entsprechend Ausführungsbeispielen die Suchstrategie (vgl. Schritt 132) entsprechend angepasst.

Das hier vorliegende Verfahren ist nicht nur auf beliebige Achssysteme und automatisiert anwendbar, sondern es ist auch nicht notwendig, das Annahmen bezüglich der Aufnahmegeometrie (d. h. vor allem hinsichtlich der verwendeten Trajektorien wie Kreis, Helix) bzw. bezüglich der Strahlgeometrie wie Kegel-, Fächer- oder Parallelstrahl getroffen werden müssen. Es ist weiter auch kein hochpräziser Referenzkörper notwendig, da dieser bei Bedarf ebenfalls innerhalb des Verfahrens kalibriert werden kann.

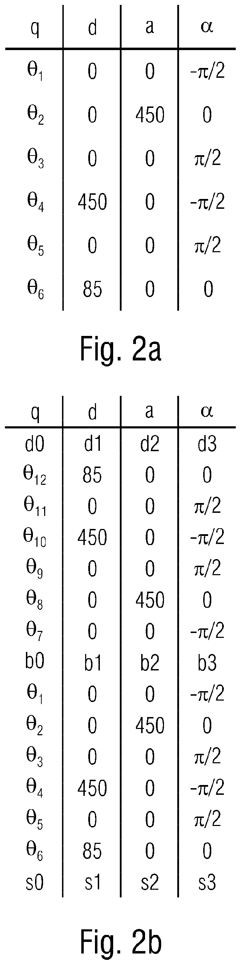

Bezug nehmend auf Fig. 2 wird nun anhand eines konkreten Beispiels das mathematische Modell erläutert. Das Verfahren wird anhand eines Systems bestehend aus zwei RX90B Robotern der Firma Stäubli sowie einem Flachbilddetektor und einer Röntgenröhre mit Kegelstrahl dargestellt.

Wie im ersten Schritt wird, wie Bezug nehmend auf Fig. 1a erläutert, das kinematische Modell aufgestellt. Ein Beispiel für ein derartiges kinematisches Modell ist in Fig. 2a dargestellt. Fig. 2a zeigt die Denavit-Hartenberg-Parameter des Roboters RX90B. Hier wird, um Bezug zu nehmen auf die Abbildung aus Fig. 1b beispielsweise der erste Roboter 52 beschrieben, der die Strahlenquelle 51s trägt. Die Distanzen sind in Millimetern angegeben, die Winkel in Abhängigkeit von p. Hierbei sei angemerkt, dass $\vartheta$ die jeweiligen Maschinenkoordinaten für die jeweiligen Gelenke 1 bis 6 sind. Die jeweilige Kette beschreibt den Anfang und das Ende des jeweiligen Manipulators.

Der erste Schritt besteht aus dem Aufstellen des kinematischen Modells des Manipulationssystems. Dazu werden die Denavit-Hartenberg Parameter für den Roboter RX90B ermittelt und diese so erweitert, dass das ganze System als eine Kinematik aufgefasst wird. Der Ursprung des Weltkoordinatensystems wird hier der Einfachheit halber mit dem Ursprung des Roboterkoordinatensystems für den Roboter mit Röntgenröhre gleichgesetzt.

Wie oben bereits erläutert, wird in demselben kinematischen Modell bzw. dem Gleichungssystem auch die Pose des zweiten Roboters mit inkludiert, so dass die Hartenberg-Parameter aus Fig. 2a um den zweiten Roboter erweitert sind und man zu den Hartenberg -Parametern aus Fig. 2b gelangt. Die Hartenberg-Parameter aus Fig. 2b beschreiben das gesamte kinemb3). Des Weiteren ist dieser Parameterdatensatz auch um den Versatz, den die Strahlungsquelle 51s, z. B. aufgrund seiner Aufhängung und der Strahlendetektor 51d, z. B. aufgrund seiner Aufhängung hat, erweitert. Hierbei ist der Parameterdatensatz um die gesuchten bzw. zu optimierenden / zu kalibrierenden Parameter für den Detektor in der Zeile mit d0 bis d3 und für den Sensor in der Zeile s0 bis s3 erweitert.

Ausgehend von diesem kinematischen Modell kann ein Gleichungssystem mit den Unbekannten d0 bis d3 bzw. b0 bis b3 bzw. s0 bis s3 aufgestellt werden, welches dann, wie oben Bezug nehmend auf Fig. 1a erläutert, gelöst werden kann.

Hierzu werden, wie bereits erläutert, die Startwerte für die zu kalibrierenden Parameter eingefügt. Startwerte für [s0, s1, s2, s3] und [d0, d1, d2, d3] werden aus den CAD Daten der Halterung entnommen. Die Startwerte für [b0, b1, b2, b3] werden mit Hilfe von Laser Entfernungsmessgeräten bestimmt.

Bei diesem Ausführungsbeispiel wird während der Kalibrierung auch noch das Projektionsmodell herangezogen.

Die Bildaufnahme mit einem Flächendetektor entspricht mathematisch einer Zentralprojektion. Alle Strahlen werden von einer Punktquelle emittiert. Das Zustandekommen der Abbildung ist durch die Lage des Projektionszentrum $\vec{s}$ und der Projektionsebene, beschrieben durch einen Ebenenaufpunkt $\vec{d}$ und den Ebenenbasisvektoren $\vec{v}$ und $\vec{w}$, definiert.

Die zur Berechnung einer Projektion benötigten Größen $\vec{s}$, $\vec{d}$, $\vec{v}$ und $\vec{w}$ können auf Basis von Maschinenkoordinaten ($\theta$) und der Vorwärtskinematik aus dem kinematischen Modell errechnet werden.

$$\begin{bmatrix} v_x & w_x & d_x - s_x \\ v_y & w_y & d_y - s_y \\ v_z & w_z & d_z - s_z \end{bmatrix} \begin{bmatrix} \lambda x' \\ \lambda y' \\ \lambda \end{bmatrix} = \begin{bmatrix} p_x - s_x \\ p_y - s_y \\ p_z - s_z \end{bmatrix}$$

Bezug nehmend auf Fig. 2 wird nun anhand eines konkreten Beispiels das mathematische Modell erläutert.

Nachfolgend werden unterschiedliche Varianten der Referenzbildung für den Kalibrier- bzw. den Referenzkörper erläutert.

Als Referenz wird der Tensor zweiten Grades (Schwerpunkt) einer Kugelabbildung verwendet. Dazu werden Röntgenprojektionen eines Kalibrierkörpers bestehend aus acht Kugeln aufgenommen. Mit Hilfe der in einer gezeigten Bildverarbeitungskette werden die benötigten Referenzdaten erzeugt. Segmentierung der Kugelabbildung und Bestimmung der Tensoren bis n-ten Grades (z. B. n = 2)

$$M_{pq} = \int\limits_{-\infty}^{\infty} \int\limits_{-\infty}^{\infty} x^p y^q f(x, y) \, dx \, dy$$

Wie bereits oben erläutert, wäre es allerdings natürlich auch denkbar, alternativ zu den Tensoren ein CAD-Modell heranzuziehen, was eine höhere Genauigkeit erwarten lässt, um so zusätzliche Aspekte aus der realen Anlage, wie z. B. die Modellierung des Brennflecks, zu betrachten.

Nachdem nun die Referenzwerte bestimmt sind, kann nun ein Fehlermaß, d. h. also die Abweichung zwischen der tatsächlichen projizierten Position und erwarteten projizierten Position ermittelt werden.

Auf Basis der Referenzwerte, der dazu gespeicherten Maschinenkoordinaten q und dem kinematischen Modell lässt sich folgendes Fehlermaß in Abhängigkeit der unbekannten Parameter u = [s0, s1, s2, s3, d0, d1, d2, d3, b0, b1, b2, b3] definieren:

$$f(\vec{u}, \vec{q}, \vec{p}) \longmapsto \vec{p_c}$$

Wobei $\vec{p}$ einen beliebigen Objektpunkt darstellt und $\vec{p_c}$ dessen Abbildung auf dem Detektor. Verfügt der Kalibrierkörper über mehrere Kugeln k und werden n Projektionen aufgenommen, gilt:

$$f(\vec{u}, \vec{q_n}, \vec{p_k}) \longmapsto \vec{p_k}'$$

Jeder Referenzpunkt kann als Lösung für diese Funktion interpretiert werden. Das Fehlermaß lautet dann:

$$c_{n,k}(\vec{u}, \vec{q}_n, \vec{p}_k) = \|f - f_{ref}\|_2$$

Eine Lösung $\vec{u}$ kann durch Lösung des Gleichungssystems

$$\min_{\vec{u}, \vec{p}} \left\| \frac{1}{NK} \sum_{n=1}^{N} \sum_{k=1}^{K} c_{n,k} \right\|_2$$

Wie oben erläutert wird eben dieses Fehlermaß der unterschiedlichen Messwerte minimiert, was im Wesentlichen eine Optimierungsaufgabe darstellt. Beispielsweise kann das Fehlermaß mithilfe des Levenberg-Marquard-Algorithmus minimiert werden, um für die gesuchten Freiheitsgrade bzw. die zu kalibrierenden Parameter eine Lösung zu bestimmen. Alternativ zum Lösen des Gleichungssystems mit der Anwendung des Optimierungsalgorithmen nach Levenberg-Marquardt (Optimierungsalgorithmen zur numerischen Lösung mit Gradientenabstieg) kann eine analytische Lösung gesucht werden oder weitere Optimierungsalgorithmen zur numerischen Lösung z.B. Gradientenabstieg oder heuristischen Algorithmen wie Downhill-Simplex angewendet werden.

Auch wenn bei obigen Ausführungsbeispielen immer von zumindest zwei angefahrenen Positionen ausgegangen wurde, sodass zwei Gleichungen über die zwei Positionen definiert werden, sei an dieser Stelle darauf hingewiesen, dass im Prinzip eine Position reicht, wenn z.B. nur ein zu kalibrierender Parameter vorliegt, oder aus der Projektionsaufnahme aus der ersten Position mehrerer Gleichungen, z.B. ausgehen von mehreren Merkmalen des Kalibrierkörpers gewonnen werden können.

Bei obigen Ausführungsbeispielen besteht zusätzlich auch noch die Möglichkeit, dass Achssysteme mit geringer Präzision justierbar sind. Ebenso sind dynamische Einflüsse während einer Aufnahme detektierbar (z. B. aufgrund von Gravitation durch unterschiedliche Stellungen eines Roboters oder aufgrund eines wandernden Brennflecks).

Entsprechend einem Ausführungsbeispiel kann anstelle oder neben der Position des Kaliberkörpers auch der Kaliberkörper selbst oder eine Geometrie desselben als zu kalibrierender Parameter festgelegt und folglich mit kalibriert werden.

Das Verfahren wird insbesondere für robotergestützte Computertomographiesysteme benötigt, da hier in der Regel zwei oder mehr Industrieroboter frei im Raum positioniert und die Röntgenkomponenten manuell an diesen befestigt werden. Zur Durchführung einer CT muss hier also die relative Lage der Roboter zueinander, sowie die Lage von Brennfleck und Abbildungsebene bekannt sein. Auch die Kalibrierung von Anlagen, bei denen Roboter zur Manipulation des zu messenden Objekts eingesetzt werden, wird hiermit ermöglicht. (FlexCT, DragonFly, etc.).

An dieser Stelle sei noch einmal darauf hingewiesen, dass die Trajektorie eines CT-Systems durch jegliche Relativbewegung zwischen Strahlenquelle/Strahlendetektor und Objekt herrühren kann. Insofern ist das oben beschriebene Verfahren auch auf jeglichem CT-System (Rotation, Helix, freibewegliche Strahlenquelle und/oder Strahlendetektor, bewegtes Objekt in Kombination mit fester Strahlenquelle/Strahlendetektor, ....) anwendbar.

Des Weiteren kann das Verfahren zur Justage von Laboranlagen eingesetzt werden, da hier ähnliche Problemstellungen bezüglich der verbauten Manipulatoren vorliegen. Insbesondere die Lage der Achsen entspricht in der Realität nicht exakt den spezifizierten Werten. Werden Röntgenkomponenten ausgetauscht, müssen solche Anlagen in der Regel aufwändig manuell nachjustiert werden. Eine Kalibrierung mit dem vorgestellten Verfahren erlaubt entweder eine einfachere Justage oder eine Rekonstruktion mit den nun bekannten Fehlergrößen. Dies kann eine sehr große Zeitersparnis für den Anwender ermöglichen.

Bei Achssystemen mit sehr geringer Genauigkeit besteht die Möglichkeit, die für die Rekonstruktion benötigten Geometrieparameter aus konkreten Positionen des Achssystems für eine konkrete Messung zu berechnen, und somit eine qualitativ hochwertige Rekonstruktion zu ermöglichen.

Dynamische Einflüsse wie die Verformung des Achssystems durch Schwerkraft bei hohem Gewicht der Röntgenkomponenten können detektiert und gegebenenfalls kompensiert werden. Auch Effekte wie eine Brennfleckwanderung während der Messung können Teil des Modells sein und mit dessen Hilfe beobachtet werden.

Auch wenn obige Ausführungsbeispiele im Zusammenhang mit ein oder mehreren Robotern, insbesondere mit mehrgliedrigen Robotern erläutert wurden, sei darauf hingewiesen, dass unter Roboter auch einfache Linearantriebe oder andere Manipulatoren zu verstehen sind.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

Literatur

UltraCal Laser-based Robot Calibration System. Robo-Technology GmbH. http://www.robo-technology.de/UltraCal_V5.pdf, Online, Stand 5. Dezember 2012.

Cheng, F. S. Calibration of robot reference frames for enhanced robot positioning accuracy. Technical report, Central Michigan University.

Heyer, T., Grigorescu, S. M., and Gräser, A. Camera calibration for reliable object manipulation in care-providing robot friend. Technical report, Institute of Automation (IAT), University of Bremen.

Hu, Z., Gui, J., Zou, J., Rong, J., Zhang, Q., Zheng, H., , and Xia, D. Geometric Calibration of a Micro-CT Systemand Performance for Insect Imaging. http://ieeexplore.ieee.org/stamp/stamp.jsp? arnumber=5871720, abgerufen am 20.05.2016.

Mery, D., Geisert, C., and Filbert, D. Geometric Calibration of a X-ray Testing System. Technische Universität Berlin. http://www.ndt. net/article/v07n03/mery02/mery02.htm, Online, Stand 12. Dezember 2012.

Stopp, F., Wieckowski, A. J., Käseberg, M., Engel, S., Fehlhaber, F., and Keeve, E. A Geometric Calibration Method for an Open Cone-Beam CT System. Charit'e Universitätsmedizin Berlin. https://www.researchgate.net/publication/236324982_A_Geometric_Calibration_Method_f or_an_Open_Cone-Beam_CT_System, Online, Stand 12. Dezember 2012. b

Sun, Y., Hou, Y., Zhao, F., and Hu, J. A Calibration Method for Misaligned Scanner Geometry in Cone-beam Computed Tomography. Technische Universität Berlin. http://www.ndt.net/article/v10n09/ yisun/yisun.pdf, Online, Stand 12. Dezember 2012.

Wang, W., Liu, F., and Yun, C. Calibration method of robot base frame using unit quaternion form. Technical report, Central Michigan University.

Yahui, G. and Xianzhong, D. (2011). Base frame calibration for coordinated industrial robots. Technical report, School of Automation, Southeast University, Nanjing, 210096, PR China.

Yang, K., Kwan, A. L. C., Miller, D. F., and Boonea, J. M. A geometric calibration method for cone beam CT systems. http://www.ncbi.nlm.nih.gov/pmc/articles/PMC2840998/, abgerufen am 20.05.2016.

Zhao, J., Hu, X., Zou, J., and Hu, X. Geometric Parameters Estimation and Calibration in Cone-Beam Micro-CT. http://www.mdpi. com/1424-8220/15/9/22811/pdf, abgerufen am 0.05.2016.

**Patentansprüche**

1. Verfahren (100, 200) zur Röntgen-basierten Kalibrierung und Justage von Achssystemen mittels eines Röntgensystems mit einer Strahlenquelle (51s) und einem Strahlendetektor (51d), mit folgenden Schritten:

> Ermitteln (110a) eines kinematischen Modells des Röntgensystems (50) mit allen kinematischen Beziehungen für eine erste Position (51t1) auf der Bewegungstrajektorie (51t) und Beschreiben derselben anhand eines ersten Kinematikparameter-Satzes und anhand von zu kalibrierenden Parametern, wobei der erste Kinematikparameter-Satz und die zu kalibrierenden Parameter eine erste Gleichung eines Gleichungssystems definieren;
> Festlegen (120) der Startwerte für die zu kalibrierenden Parameter; und
> Kalibrieren (130) mit folgenden Unterschritten:
>
>> Ermitteln (132a) einer Röntgenprojektion eines Kalibrierkörpers (60) für die erste Position (51t1) auf der Bewegungstrajektorie (51t), um für zumindest ein Merkmal des Kalibrierkörpers (60) ein erstes Messergebnis zu erhalten; und
>> Vergleichen (134a) des ersten Messergebnisses mit einer jeweiligen Referenz, um ein Fehlermaß zu bestimmen; und
>> Lösen des Gleichungssystems, um die zu kalibrierenden Parameter zu bestimmen;
>
> wobei das Röntgensystem zumindest einen Roboter oder einen mehrgliedrigen Roboter (52, 54) umfasst; wobei der Roboter oder der mehrgliedrige Roboter die Strahlenquelle (51s), ein Objekt oder den Detektor bewegt, so dass die Strahlenquelle (51s), das Objekt und/oder der Strahlendetektor (51d) in Abhängigkeit von Maschinenparametern zur Roboteransteuerung bewegbar sind; und
> wobei die Kinematikparameter des Kinematikparametersatzes das kinematische Modell des Gesamtsystems, umfassend zumindest den Robotor oder den mehrgliedrigen Roboter (52, 54), beschreiben, wobei die Kinematikparameter nicht veränderliche Kinematikparameter, umfassend eine Schenkellänge einer Komponente des Robotors oder des mehrgliedrigen Roboters (52, 54), und veränderliche Kinematikparameter, umfassend die Maschinenparameter, die die Bewegung oder Position des jeweiligen Gelenks des Robotors oder des mehrgliedrigen Roboters (52, 54) beschreiben, umfasst.

**2.** Verfahren (100, 200) gemäß Anspruch 1, wobei beim Lösen des Gleichungssystems zu kalibrierenden Parameter, welche für zumindest zwei oder alle Punkte auf der Bewegungstrajektorie (51t) gültig sind, bestimmt werden.

**3.** Verfahren (100, 200) gemäß einem der vorherigen Ansprüche, wobei die Schritte Ermitteln (110b) eines kinematischen Modells des Röntgensystems (50) mit allen kinematischen Beziehungen für eine zweite Position (51t2) auf der Bewegungstrajektorie (51t) wiederholt werden, um alle kinematischen Beziehungen für eine zweite Position (51t2) mit einem zweiten Kinematikparameter-Satz und anhand von den zu kalibrierende Parametern zu beschreiben, wobei der zweite Kinematikparameter-Satz und die zu kalibrierende Parametern eine zweite Gleichung des Gleichungssystems definieren; und wobei das Kalibrieren (130) die folgenden Unterschritten umfasst:

Ermitteln (132b) einer Röntgenprojektion des Kalibrierkörpers (60) für die zweite Position (51t2) auf der Bewegungstrajektorie (51t), um für zumindest ein Merkmal des Kalibrierkörpers (60) ein zweites Messergebnis zu erhalten; und Vergleichen (134b) des zweiten Messergebnisses mit einer jeweiligen Referenz, um ein Fehlermaß erneut zu bestimmen.

**4.** Verfahren (100, 200) gemäß einem der vorherigen Ansprüche, wobei jeder erste und der zweite Kinematikparameter-Satz sich hinsichtlich der nicht veränderlichen Kinematikparameter gleichen und sich hinsichtlich der Maschinenparameter, die eine Gelenkposition für den jeweiligen Freiheitsgrad definieren, unterscheiden; und wobei die zu kalibrierenden Parameter über die Kinematikparameter-Sätze konstant sind; und/oder wobei jeder Kinematikparameter-Satz anhand Minimaldarstellungen, Minimaldarstellungen nach Denavit-Hartenberg oder nach Hayati-Roberts Modell definierbar ist.

**5.** Verfahren (100, 200) gemäß einem der vorherigen Ansprüche, wobei das Röntgensystem zwei mehrgliedrige Roboter umfasst; wobei ein erster der zwei Roboter die Strahlenquelle (51s) bewegt und der zweite der zwei Roboter den Strahlendetektor (51d) bewegt, so dass die Strahlenquelle (51s) und/oder der Strahlendetektor (51d) in Abhängigkeit von Maschinenparametern zur Robotersteuerung bewegbar sind.

**6.** Verfahren (100, 200) gemäß Anspruch 5, wobei die zu kalibrierenden Parameter einen Versatz der zwei Roboter beschreiben; und/oder wobei die zu kalibrierenden Parameter einen Versatz der Strahlenquelle (51s) gegenüber dem in dem kinematischen Modell beschriebenen Roboter, der die Strahlenquelle (51s) bewegt, beschreiben; und/oder wobei die zu kalibrierenden Parameter einen Versatz zwischen dem Röntgendetektor und dem kinematischen Modell des Roboters, der den Strahlendetektor (51d) bewegt, beschreiben.

**7.** Verfahren (100, 200) gemäß einem der vorherigen Ansprüche, wobei das kinematische Modell alle Beziehungen für die Bewegung der Strahlenquelle (51s), für die Bewegung des Strahlendetektors (51d) und für die Position des Kalibrierobjekts im Raum umfasst; oder wobei das kinematische Modell alle Beziehungen für die Bewegung der Strahlenquelle (51s), für die Bewegung des Strahlendetektors (51d) und für die Position des Kalibrierobjekts im Raum umfasst und wobei die die Position des Kalibrierobjekts im Raum beschreibenden Parameter zumindest einen zu kalibrierenden Parameter aufweisen; wobei das kinematische Modell alle Beziehungen für die Bewegung der Strahlenquelle (51s), für die Bewegung des Strahlendetektors (51d) und für die Position des Kalibrierobjekts im Raum umfasst und wobei die die Position des Kalibrierobjekts im Raum beschreibenden Parameter zumindest einen zu kalibrierenden Parameter aufweisen; und/oder wobei die die Geometrie des Kalibrierobjekts beschreibenden Parameter zumindest einen zu kalibrierenden Parameter umfassen.

**8.** Verfahren (100, 200) gemäß einem der vorherigen Ansprüche, wobei das Gleichungssystem anhand von

$$\min_{\vec{u},\vec{p}} \| \frac{1}{NK} \sum_{n=1}^{N} \sum_{k=1}^{K} e_{n,k} \|_2 \, ,$$

mit dem Fehlermaß

$$e_{n,k}(\vec{u}, \vec{q}_n, \vec{p}_k) = \| f - f_{ref} \|_2$$

definiert ist;

wobei $u$ die zu kalibrierenden Parameter beschreibt und $f(\vec{u}, \vec{q}, \vec{p}) \rightarrow \vec{p}_c$ eine Abbildungsfunktion, wenn nur ein Merkmal des Kalibrierobjekts verwendet wird, oder u die zu kalibrierenden Parameter beschreibt und $f(\vec{u}, \vec{q}_n, \vec{p}_k) \rightarrow \vec{p}_k'$ eine Abbildungsfunktion, wenn mehrere Merkmale des Kalibrierkörpers durch mehrere Projektionen ermittelt werden,

wobei q die jeweilige Referenz mit den Koordinaten des kinematischen Modells beschreibt und wobei p das Merkmal des Kalibrierkörpers für das erste und/oder das zweite Messergebnis mit dem Koordinatensystem des kinematischen Modells beschreibt, wobei $p_k$ die Merkmale des Kalibrierkörpers für das erste und/oder zweite Messergebnis in den Koordinaten des kinematischen Modells beschreibt und wobei p' die Abbildung des Merkmals auf den Detektor mit den Koordinaten des kinematischen Modells beschreibt, und $p_k'$ die Abbildung des jeweiligen Merkmals auf den Detektor mit den Koordinaten des kinematischen Modells beschreibt.

9. Verfahren (100, 200) gemäß einem der vorherigen Ansprüche, wobei das Lösen des Gleichungssystems den Unterschritt einer analytischen Lösung oder der Anwendung eines Optimierungsalgorithmus nach Levenberg-Marquardt oder eines heuristischen Algorithmus nach Downhill-Simplex umfasst.

10. Verfahren (100, 200) gemäß einem der vorherigen Ansprüche, wobei der Schritt des Festlegens der Startwerte den Unterschritt des messtechnischen Ermittelns einer Beziehung zwischen den zwei Roboterursprüngen, des Bestimmens einer Geometrie der Halterung für die Strahlenquelle (51s) und/oder des Bestimmens einer Geometrie einer Halterung für den Strahlendetektor (51 d) umfasst.

11. Verfahren (100, 200) gemäß einem der Ansprüche 1 bis 10, wobei der Kalibrierkörper (60) bekannt ist und die jeweilige Referenz rechentechnisch ermittelt wird.

12. Verfahren (100, 200) gemäß einem der vorherigen Ansprüche, wobei beim Kalibrieren das Projektionsmodell des jeweiligen Strahlenquellen-Strahlendetektor-Paars mit berücksichtigt wird; oder
wobei beim Kalibrieren das Projektionsmodell des jeweiligen Strahlenquellen-Strahlendetektor-Paars mit berücksichtigt wird und wobei das Projektionsmodell durch folgende Formel definiert ist:

$$\begin{bmatrix} v_x & w_x & d_x - s_x \\ v_y & w_y & d_y - s_y \\ v_z & w_z & d_z - s_z \end{bmatrix} \begin{bmatrix} \lambda x' \\ \lambda y' \\ \lambda \end{bmatrix} = \begin{bmatrix} p_x - s_x \\ p_y - s_y \\ p_z - s_z \end{bmatrix},$$

wobei $p_x$, $p_y$ und $p_z$ die Position des Merkmals des Kalibrierkörpers (60) im Raum beschreibt, wobei $s_x$, $s_y$ und $s_z$ die Position des Projektionszentrums im Raum beschreibt, wobei $d_x$, $d_y$ und $d_z$ die Position der Referenz im Raum beschreibt, wobei $v_x$, $v_y$ und $v_z$ sowie $\omega_x$, $\omega_y$ und $\omega_z$ die ebenen Basisvektoren sind, wobei s, d, v und $\omega$ mit den Koordinaten des kinematischen Modells beschrieben sind.

13. Computerprogramm mit einem Programmcode zur Durchführung des Verfahrens nach einem der vorherigen Ansprüche, wenn das Programm auf einem Computer eines Röntgensystems laut Anspruch 1 abläuft.

14. Vorrichtung zur Röntgen-basierten Kalibrierung und Justage von Achssystemen mittels eines Röntgensystems mit einer Strahlenquelle (51s) und einem Strahlendetektor (51d), mit folgenden Merkmalen:

eine Berechnungseinheit zum Ermitteln eines kinematischen Modells des Röntgensystems (50) mit allen kinematischen Beziehungen für eine erste Position (51t1) auf der Bewegungstrajektorie (51t) und Beschreiben desselben anhand eines ersten Kinematikparameter-Satzes und anhand von zu kalibrierenden Parameter, wobei der erste Kinematikparameter-Satz und die zu kalibrierenden Parameter eine erste Gleichung eines Gleichungssystems definieren, einem Kalibrierer, der ausgehend von festgelegten Startwerten für die zu kalibrierenden Parameter ausgebildet ist zum:

Ermitteln einer Röntgenprojektion eines Kalibrierkörpers (60) für die erste Position (51t1) auf der Bewegungstrajektorie (51t), um für zumindest ein Merkmal des Kalibrierkörpers (60) ein erstes Messergebnis zu erhalten; und
Vergleichen des ersten Messergebnisses mit einer jeweiligen Referenz, um ein Fehlermaß zu bestimmen;
wobei der Kalibrierer ausgebildet ist, das Gleichungssystem zu lösen, um die zu kalibrierenden Parameter zu erhalten;
wobei das Röntgensystem zumindest einen Roboter oder einen mehrgliedrigen Roboter (52, 54) umfasst; wobei der Roboter oder der mehrgliedrige Roboter die Strahlenquelle (51s), ein Objekt oder den Detektor bewegt, so dass die Strahlenquelle (51s), das Objekt und/oder der Strahlendetektor (51d) in Abhängigkeit von Maschinenparametern zur Robotersteuerung bewegbar sind; und
wobei die Kinematikparameter des Kinematikparametersatzes das kinematische Modell des Gesamtsystems, umfassend zumindest den Roboter oder den mehrgliedrigen Roboter (52, 54), beschreiben, wobei die Kinematikparameter nicht veränderliche Kinematikparameter, umfassend eine Schenkellänge einer Komponente des Robotors oder des mehrgliedrigen Roboters (52, 54), und veränderliche Kinematikparameter, umfassend die Maschinenparameter, die die Bewegung oder Position des jeweiligen Gelenks des Robotors oder des mehrgliedrigen Roboters (52, 54) beschreiben, umfasst.

15. Vorrichtung gemäß Anspruch 14, wobei die Vorrichtung eine Schnittstelle zum Steuern des Röntgensystems (50) und/oder zum Steuern von Robotern des Röntgensystems umfasst.

16. Röntgensystem (50) mit einer Strahlenquelle (51s) und einer Röntgenquelle sowie einer Vorrichtung gemäß einem der Ansprüche 14 oder 15.

17. Röntgensystem (50) gemäß Anspruch 16, wobei das Röntgensystem einen ersten Roboter, mittels welchem die Strahlenquelle (51s) bewegbar ist, und einen zweiten Roboter, mittels welchem der Strahlendetektor (51 d) bewegbar ist, umfasst.

**Claims**

1. A method (100, 200) for an X-ray-based calibration and adjustment of axis systems by means of an X-ray system comprising a radiation source (51s) and a radiation detector (51d), comprising:

   establishing (110a) a kinematic model of the X-ray system (50) with all the kinematic relations for a first position (51t1) on the movement trajectory (51t) and describing the same using a first set of kinematic parameters and using parameters to be calibrated, wherein the first set of kinematic parameters and the parameters to be calibrated define a first equation of a system of equations;
   setting (120) the starting values for the parameters to be calibrated; and
   calibrating (130) comprising:

   establishing (132a) an X-ray projection of a calibration body (60) for the first position (51t1) on the movement trajectory (51t) in order to acquire a first measuring result for at least one feature of the calibration body (60); and
   comparing (134a) the first measuring result to a respective reference in order to determine an error measure; and
   solving the system of equations in order to determine the parameters to be calibrated;

   wherein the X-ray system comprises at least one robot or multi-element robot (52, 54); wherein the robot or multi-element robot moves the radiation source (51s), an object or the detector such that the radiation source (51s), the object and/or the radiation detector (51d) are movable in dependence on machine parameters for driving the robot; and
wherein the kinematic parameters of the set of kinematic parameters describe the kinematic model of the entire system comprising at least the robot or multi-element robot (52, 54), wherein the kinematic parameters comprise non-variable kinematic parameters comprising a leg length of a component of the robot or multi-element robot (52, 54), and variable kinematic parameters comprising the machine parameters which describe the movement or position of the respective joint of the robot or multi-element robot (52, 54).

2. The method (100, 200) in accordance with claim 1, wherein parameters to be calibrated which are valid for at least two or all of the points on the movement trajectory (51t) are determined when solving the system of equations.

3. The method (100, 200) in accordance with any of the preceding claims, wherein establishing (110b) a kinematic model of the X-ray system (50) with all the kinematic relations is repeated for a second position (51t2) on the movement trajectory (51t) in order to describe all the kinematic relations for a second position (51t2) with a second set of kinematic parameters and using the parameters to be calibrated, wherein the second set of kinematic parameters and the parameters to be calibrated define a second equation of the system of equations; and wherein calibrating (130) comprises:

   establishing (132b) an X-ray projection of the calibration body (60) for the second position (51t2) on the movement trajectory (51t) in order to acquire a second measuring result for at least one feature of the calibration body (60); and
   comparing (134b) the second measuring result to a respective reference in order to re-determine an error measure.

4. The method (100, 200) in accordance with any of the preceding claims, wherein each first and the second set of kinematic parameters are equal relative to the non-variable kinematic parameters and differ relative to the machine parameters which define a joint position for the respective degree of freedom; and
wherein the parameters to be calibrated are constant over the sets of kinematic parameters; and/or
wherein each set of kinematic parameters is definable using minimum representations, minimum representations in accordance with Denavit-Hartenberg or in accordance with the Hayati-Roberts model.

5. The method (100, 200) in accordance with any of the preceding claims, wherein the X-ray system comprises two multi-element robots;
wherein a first one of the two robots moves the radiation source (51s) and the second one of the two robots moves the radiation detector (51d) so that the radiation source (51s) and/or the radiation detector (51d) is/are movable in dependence on machine parameters for driving the robots.

6. The method (100, 200) in accordance with claim 5, wherein the parameters to be calibrated describe an offset of the two robots; and/or
wherein the parameters to be calibrated describe an offset of the radiation source (51s) relative to the robot, described in the kinematic model, which moves the radiation source (51s); and/or
wherein the parameters to be calibrated describe an offset between the X-ray detector and the kinematic model of the robot which moves the radiation detector (51 d).

7. The method (100, 200) in accordance with any of the preceding claims, wherein the kinematic model comprises all the relations for moving the radiation source (51s), for moving the radiation detector (51d) and for the position of the calibration object in space; or
wherein the kinematic model comprises all the relations for moving the radiation source (51s), for moving the radiation detector (51d) and for the position of the calibration object in space and wherein the parameters describing the position of the calibration object in space comprise at least one parameter to be calibrated;
wherein the kinematic model comprises all the relations for moving the radiation source (51s), for moving the radiation detector (51d) and for the position of the calibration object in space and wherein the parameters describing the position of the calibration object in space comprise at least one parameter to be calibrated; and/or wherein the parameters describing the geometry of the calibration object comprise at least one parameter to be calibrated.

8. The method (100, 200) in accordance with any of the preceding claims, wherein the system of equations is defined by:

$$\min_{\vec{u},\vec{p}} \left\| \frac{1}{NK} \sum_{n=1}^{N} \sum_{k=1}^{K} e_{n,k} \right\|_2 ,$$

with the following error measure:

$$e_{n,k}(\vec{u}, \vec{q_n}, \vec{p_k}) = \| f - f_{ref} \|_2 ,$$

wherein $u$ describes the parameters to be calibrated and $f(\vec{u}, \vec{q}, \vec{p}) \rightarrow \vec{p_c}$ describes a mapping function when using only one feature of the calibration object,
or $u$ describes the parameters to be calibrated and describes a mapping function when several features of the calibration body are established by means of several projections,
wherein q describes the respective reference with the coordinates of the kinematic model, and wherein p describes the feature of the calibration body for the first and/or the second measuring result with the coordinate system of the kinematic model, wherein $p_k$ describes the features of the calibration body for the first and/or second measuring result in the coordinates of the kinematic model, and wherein p' describes mapping the feature on the detector with the coordinates of the kinematic model, and $p_k$' describes mapping of the respective feature on the detector with the coordinates of the kinematic model.

9. The method (100, 200) in accordance with any of the preceding claims, wherein solving the system of equations comprises analytically solving or applying an optimizing algorithm in accordance with Levenberg-Marquardt or a heuristic algorithm in accordance with Downhill-Simplex.

10. The method (100, 200) in accordance with any of the preceding claims, wherein setting the starting values comprises establishing, by means of measuring technology, a relation between the two robot origins, determining a geometry of the holder for the radiation source (51s) and/or determining a geometry of a holder for the radiation detector (51d).

11. The method (100, 200) in accordance with any of claims 1 to 10, wherein the calibration body (60) is known and the respective reference is established by means of calculating.

12. The method (100, 200) in accordance with any of the preceding claims, wherein the projection model of the respective pair of radiation source and radiation detector is considered when calibrating and wherein the projection model is defined by the following formula:

$$\begin{bmatrix} v_x & w_x & d_x - s_x \\ v_y & w_y & d_y - s_y \\ v_z & w_z & d_z - s_z \end{bmatrix} \begin{bmatrix} \lambda x' \\ \lambda y' \\ \lambda \end{bmatrix} = \begin{bmatrix} p_x - s_x \\ p_y - s_y \\ p_z - s_z \end{bmatrix},$$

wherein $p_x$, $p_y$ and $p_z$ describe the position of the feature of the calibration body (60) in space, wherein $s_x$, $s_y$ and $s_z$ describe the position of the projection center in space, wherein $d_x$, $d_y$ and $d_z$ describe the position of the reference in space, wherein $v_x$, $v_y$ and $v_z$ and $\omega_x$, $\omega_y$ and $\omega_z$ are the planar basis vectors, wherein s, d, v and $\omega$ are described by the coordinates of the kinematic model.

13. A computer program having stored theron a program code for performing the method in accordance with any of the preceding claims, when said computer program is run by a computer of an X-ray system in accordance with claim 1.

14. An apparatus for an X-ray-based calibration and adjustment of axis systems by means of an X-ray system comprising a radiation source (51s) and a radiation detector (51d), comprising:

a calculation unit for establishing a kinematic model of the X-ray system (50) with all the kinematic relations for a first position (51t1) on the movement trajectory (51t) and describing the same using a first set of kinematic parameters and using parameters to be calibrated, wherein the first set of kinematic parameters and the parameters to be calibrated define a first equation of a system of equations, a calibrator configured, based on fixed starting values for the parameters to be calibrated, to:

establish an X-ray projection of a calibration body (60) for the first position (51t1) on the movement trajectory (51t) in order to acquire a first measuring result for at least one feature of the calibration body (60); and comparing the first measuring result to a respective reference in order to determine an error measure; wherein the calibrator is configured to solve the system of equations in order to acquire the parameters to be calibrated; wherein the X-ray system comprises at least one robot or multi-element robot (52, 54); wherein the robot or multi-element robot moves the radiation source (51s), an object or the detector such that the radiation source (51s), the object and/or the radiation detector (51d) are movable in dependence on machine parameters for driving the robot; and

wherein the kinematic parameters of the set of kinematic parameters describe the kinematic model of the entire system comprising at least the robot or multi-element robot (52, 54), wherein the kinematic parameters comprise non-variable kinematic parameters comprising a leg length of a component of the robot or multi-element robot (52, 54), and variable kinematic parameters comprising the machine parameters which describe the movement or position of the respective joint of the robot or multi-element robot (52, 54).

15. The apparatus in accordance with claim 14, the apparatus comprising an interface for controlling the X-ray system (50) and/or for controlling robots of the X-ray system.

16. An X-ray system (50) comprising a radiation source (51s) and an X-ray source, and an apparatus in accordance with any of claims 14 or 15.

17. The X-ray system (50) in accordance with claim 16, wherein the X-ray system comprises a first robot by means of which the radiation source (51s) is movable, and a second robot by means of which the radiation detector (51d) is movable.

**Revendications**

1. Procédé (100, 200) d'étalonnage et d'ajustement à base de rayons X de systèmes d'axes au moyen d'un système radiographique avec une source de rayonnement (51s) et un détecteur de rayonnement (51d), aux étapes suivantes consistant à:

déterminer (110a) un modèle cinématique du système radiographique (50) avec tous les rapports cinématiques pour une première position (51t1) sur la trajectoire de déplacement (51t) et décrire ces derniers à l'aide d'un premier ensemble de paramètres cinématiques et à l'aide de paramètres à étalonner, où le premier ensemble de paramètres cinématiques et les paramètres à étalonner définissent une première équation d'un système d'équations; fixer (120) les valeurs de départ pour les paramètres à étalonner; et étalonner (130) avec les sous-étapes suivantes consistant à:

déterminer (132a) une projection sous rayons X d'un corps d'étalonnage (60) pour la première position (51t1) sur la trajectoire de déplacement (51t) pour obtenir, pour au moins une caractéristique du corps d'étalonnage (60), un premier résultat de mesure; et

comparer (134a) le premier résultat de mesure avec une référence respective pour déterminer une mesure d'erreur; et

résoudre le système d'équations pour déterminer les paramètres à étalonner;

dans lequel le système radiographique comporte au moins un robot ou un robot à plusieurs éléments (52, 54); dans lequel le robot ou le robot à plusieurs éléments déplace la source de rayonnement (51s), un objet ou le détecteur, de sorte que la source de rayonnement (51s), l'objet et/ou le détecteur de rayonnement (51d) puissent être déplacés en fonction des paramètres de machine pour la commande du robot; et

dans lequel les paramètres cinématiques de l'ensemble de paramètres cinématiques décrivent le modèle cinématique du système global, comportant au moins le robot ou le robot à plusieurs éléments (52, 54), dans lequel les paramètres cinématiques comportent des paramètres cinématiques non variables comportant une longueur de branche d'un composant du robot ou du robot à plusieurs éléments (52, 54), et des paramètres cinématiques variables comportant les paramètres de machine qui décrivent le déplacement ou la position de l'articulation respective du robot ou du robot à plusieurs éléments (52, 54).

2. Procédé (100, 200) selon la revendication 1, dans lequel sont déterminés, lors de la résolution du système d'équations, les paramètres à étalonner qui sont valables pour au moins deux ou pour tous les points sur la trajectoire de déplacement (51t).

3. Procédé (100, 200) selon l'une des revendications précédentes, dans lequel les étapes consistant à déterminer (110b) un modèle cinématique du système radiographique (50) sont répétées avec tous les rapports cinématiques pour une deuxième position (51t2) sur la trajectoire de déplacement (51t) pour décrire tous les rapports cinématiques pour une deuxième position (51t2) avec un deuxième ensemble de paramètres cinématiques et à l'aide des paramètres à étalonner, dans lequel le deuxième ensemble de paramètres cinématiques et les paramètres à étalonner définissent une deuxième équation du système d'équations; et dans lequel l'étalonnage (130) comporte les sous-étapes suivantes consistant à:

déterminer (132b) une projection sous rayons X du corps d'étalonnage (60) pour la deuxième position (51t2) sur la trajectoire de déplacement (51t) pour obtenir, pour au moins une caractéristique du corps d'étalonnage (60), un deuxième résultat de mesure; et

comparer (134b) le deuxième résultat de mesure avec une référence respective pour déterminer à nouveau une mesure d'erreur.

4. Procédé (100, 200) selon l'une des revendications précédentes, dans lequel chaque premier et le deuxième ensemble de paramètres cinématiques sont identiques en ce qui concerne les paramètres cinématiques non variables et diffèrent en ce qui concerne les paramètres de machine qui définissent une position d'articulation pour le degré de liberté respectif; et

dans lequel les paramètres à étalonner sont constants sur les jeux de paramètres cinématiques; et/ou dans lequel chaque ensemble de paramètres cinématiques peut être défini à l'aide de représentations minimales, de représentations minimales selon Denavit-Hartenberg ou selon le modèle de Hayati-Roberts.

5. Procédé (100, 200) selon l'une des revendications précédentes, dans lequel le système radiographique comporte deux robots à plusieurs éléments; dans lequel un premier des deux robots déplace la source de rayonnement (51s) et le deuxième des deux robots déplace le détecteur de rayonnement (51d), de sorte que la source de rayonnement (51s) et/ou le détecteur de rayonnement (51d) puissent être déplacés en fonction des paramètres de machine pour la commande du robot.

6. Procédé (100, 200) selon la revendication 5, dans lequel les paramètres à étalonner décrivent un décalage des deux robots; et/ou dans lequel les paramètres à étalonner décrivent un décalage de la source de rayonnement (51s) par rapport au robot décrit dans le modèle cinématique qui déplace la source de rayonnement (51s); et/ou dans lequel les paramètres à étalonner décrivent un décalage entre le détecteur de rayons X et le modèle cinématique du robot qui déplace le détecteur de rayonnement (51d).

7. Procédé (100, 200) selon l'une des revendications précédentes, dans lequel le modèle cinématique comporte tous les rapports pour le déplacement de la source de rayonnement (51s), pour le déplacement du détecteur de rayonnement (51 d) et pour la position de l'objet d'étalonnage dans l'espace; ou dans lequel le modèle cinématique comporte tous les rapports pour le déplacement de la source de rayonnement (51s), pour le déplacement du détecteur de rayonnement (51d) et pour la position de l'objet d'étalonnage dans l'espace, et dans lequel les paramètres décrivant la position de l'objet d'étalonnage dans l'espace présentent au moins un paramètre à étalonner;

dans lequel le modèle cinématique comporte tous les rapports pour le déplacement de la source de rayonnement (51s), pour le déplacement du détecteur de rayonnement (51d) et pour la position de l'objet d'étalonnage dans l'espace, et dans lequel les paramètres décrivant la position de l'objet d'étalonnage dans l'espace présentent au moins un paramètre à étalonner; et/ou dans lequel les paramètres décrivant la géométrie de l'objet d'étalonnage comportent au moins un paramètre à étalonner.

8. Procédé (100, 200) selon l'une des revendications précédentes, dans lequel le système d'équations est défini à l'aide de

$$\min_{\vec{u}\,\vec{p}} \left\| \frac{1}{NK} \sum_{n=1}^{N} \sum_{k=1}^{K} e_{n,k} \right\| 2 ,$$

avec la mesure d'erreur

$$e_{n,k}(\vec{u}, \vec{q}_n, \vec{p}_k) = \left\| f - f_{ref} \right\|_2 ;$$

où *u* décrit les paramètres à étalonner et $f(\vec{u}, \vec{q}, \vec{p}) \rightarrow \vec{p}_e$ décrit une fonction de reproduction lorsqu'il est utilisé une seule caractéristique de l'objet d'étalonnage, ou *u* décrit les paramètres à étalonner et $f(\vec{u}, \vec{q}_n, \vec{p}_k) \rightarrow \vec{p}_k'$ décrit une fonction de reproduction lorsque plusieurs caractéristiques du corps d'étalonnage sont déterminées par plusieurs projections,

où q décrit la référence respective avec les coordonnées du modèle cinématique et où p décrit la caractéristique du corps d'étalonnage pour le premier et/ou le deuxième résultat de mesure avec le système de coordonnées du modèle cinématique, où $p_k$ décrit les caractéristiques du corps d'étalonnage pour le premier et/ou le deuxième résultat de mesure dans les coordonnées du modèle cinématique et où p' décrit la reproduction de la caractéristique sur le détecteur avec les coordonnées du modèle cinématique, et $p_k'$ décrit la reproduction de la caractéristique respective sur le détecteur avec les coordonnées du modèle cinématique.

9. Procédé (100, 200) selon l'une des revendications précédentes, dans lequel la résolution du système d'équation comporte la sous-étape consistant à résoudre de manière analytique ou à appliquer un algorithme d'optimisation selon Levenberg-Marquardt ou un algorithme heuristique selon Downhill-Simplex.

10. Procédé (100, 200) selon l'une des revendications précédentes, dans lequel l'étape consistant à fixer les valeurs de départ comporte la sous-étape consistant à déterminer par la technique de mesure un rapport entre les deux origines de robot, à déterminer une géométrie du support pour la source de rayonnement (51s) et/ou à déterminer une géométrie d'un support pour le détecteur de rayonnement (51d).

11. Procédé (100, 200) selon l'une des revendications 1 à 10, dans lequel le corps d'étalonnage (60) est connu et la référence respective est déterminée par la technique de calcul.

12. Procédé (100, 200) selon l'une des revendications précédentes, dans lequel il est tenu compte, lors de l'étalonnage, du modèle de projection de la paire respective de source de rayonnement et détecteur de rayonnement; ou dans lequel il est tenu compte, lors de l'étalonnage, du modèle de projection de la paire respective de source de rayonnement et détecteur de rayonnement et dans lequel le modèle de projection est défini par la formule suivante:

$$\begin{bmatrix} v_x & w_x & d_x - s_x \\ v_y & w_y & d_y - s_y \\ v_z & w_z & d_z - s_z \end{bmatrix} \begin{bmatrix} \lambda x' \\ \lambda y' \\ \lambda \end{bmatrix} = \begin{bmatrix} p_x - s_z \\ p_y - s_z \\ p_z - s_z \end{bmatrix},$$

où $p_x$, $p_y$ et $p_z$ décrivent la position de la caractéristique du corps d'étalonnage (60) dans l'espace, où $s_x$, $s_y$ et $s_z$ décrivent la position du centre de projection dans l'espace, où $d_x$, $d_y$ et $d_z$ décrivent la position de la référence dans l'espace, où $v_x$, $v_y$ et $v_z$ ainsi que $\omega_x$, $\omega_y$ et $\omega_z$ sont des vecteurs de base plans,
où s, d, v et $\omega$ sont décrits avec les coordonnées du modèle cinématique.

13. Programme d'ordinateur avec un code de programme pour réaliser le procédé selon l'une des revendications précédentes lorsque le programme est exécuté sur un ordinateur d'un système radiographique selon la revendication 1.

14. Dispositif d'étalonnage et de réglage à base de rayons X de systèmes d'axes au moyen d'un système radiographique avec une source de rayonnement (51s) et un détecteur de rayonnement (51d), aux caractéristiques suivantes:

une unité de calcul destinée à déterminer un modèle cinématique du système radiographique (50) avec tous les rapports cinématiques pour une première position (51t1) sur la trajectoire de déplacement (51t) et à décrire ces derniers à l'aide d'un premier ensemble de paramètres cinématiques et à l'aide de paramètres à étalonner, où le premier ensemble de paramètres cinématiques et les paramètres à étalonner définissent une première équation d'un système d'équations,

un étalonneur qui, partant de valeurs de départ fixées pour les paramètres à étalonner, est conçu pour:

déterminer une projection sous rayons X d'un corps d'étalonnage (60) pour la première position (51t1) sur la trajectoire de déplacement (51t) pour obtenir, pour au moins une caractéristique du corps d'étalonnage (60), un premier résultat de mesure; et comparer le premier résultat de mesure avec une référence respective pour déterminer une mesure d'erreur;

dans lequel l'étalonneur est conçu pour résoudre le système d'équations pour obtenir les paramètres à étalonner;

dans lequel le système radiographique comporte au moins un robot ou un robot à plusieurs éléments (52, 54); dans lequel le robot ou le robot à plusieurs éléments déplace la source de rayonnement (51s), un objet ou le détecteur, de sorte que la source de rayonnement (51s), l'objet et/ou le détecteur de rayonnement (51d) puissent être déplacés en fonction des paramètres de machine pour la commande du robot; et

dans lequel les paramètres cinématiques de l'ensemble de paramètres cinématiques décrivent le modèle cinématique du système global, comportant au moins le robot ou le robot à plusieurs éléments (52, 54), dans lequel les paramètres cinématiques comportent des paramètres cinématiques non variables comportant une longueur de branche d'un composant du robot ou du robot à plusieurs éléments (52, 54), et des paramètres cinématiques variables comportant les paramètres de machine qui décrivent le déplacement ou la position de l'articulation respective du robot ou du robot à plusieurs éléments (52, 54).

15. Dispositif selon la revendication 14, dans lequel le dispositif comporte une interface destinée à commander le système radiographique (50) et/ou à commander des robots du système radiographique.

16. Système radiographique (50) avec une source de rayonnement (51s) et une source de rayons X ainsi qu'un dispositif selon l'une des revendications 14 ou 15.

17. Système radiographique (50) selon la revendication 16, dans lequel le système radiographique comporte un premier robot au moyen duquel peut être déplacée la source de rayonnement (51s), et un deuxième robot au moyen duquel peut être déplacé le détecteur de rayonnement (51d).

100

110a

110b

120

130

132a

132b

134a

134b

140

Fig. 1a

Fig. 1b

Fig. 1c

| q | d | a | α |
|---|---|---|---|
| $\theta_1$ | 0 | 0 | $-\pi/2$ |
| $\theta_2$ | 0 | 450 | 0 |
| $\theta_3$ | 0 | 0 | $\pi/2$ |
| $\theta_4$ | 450 | 0 | $-\pi/2$ |
| $\theta_5$ | 0 | 0 | $\pi/2$ |
| $\theta_6$ | 85 | 0 | 0 |

## Fig. 2a

| q | d | a | α |
|---|---|---|---|
| d0 | d1 | d2 | d3 |
| $\theta_{12}$ | 85 | 0 | 0 |
| $\theta_{11}$ | 0 | 0 | $\pi/2$ |
| $\theta_{10}$ | 450 | 0 | $-\pi/2$ |
| $\theta_9$ | 0 | 0 | $\pi/2$ |
| $\theta_8$ | 0 | 450 | 0 |
| $\theta_7$ | 0 | 0 | $-\pi/2$ |
| b0 | b1 | b2 | b3 |
| $\theta_1$ | 0 | 0 | $-\pi/2$ |
| $\theta_2$ | 0 | 450 | 0 |
| $\theta_3$ | 0 | 0 | $\pi/2$ |
| $\theta_4$ | 450 | 0 | $-\pi/2$ |
| $\theta_5$ | 0 | 0 | $\pi/2$ |
| $\theta_6$ | 85 | 0 | 0 |
| s0 | s1 | s2 | s3 |

## Fig. 2b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015051468 A

**In der Beschreibung aufgeführte Nicht-Patentliteratur**

- UltraCal Laser-based Robot Calibration System. *Robo-Technology GmbH,* Dezember 2012, http://www.robo-technology.de/UltraCal_V5.pdf
- Calibration of robot reference frames for enhanced robot positioning accuracy. **CHENG, F. S.** Technical report. Central Michigan University
- Camera calibration for reliable object manipulation in care-providing robot friend. **HEYER, T. ; GRIGOR-ESCU, S. M. ; GRÄSER, A.** Technical report. Institute of Automation (IAT)
- **HU, Z. ; GUI, J. ; ZOU, J. ; RONG, J. ; ZHANG, Q. ; ZHENG, H. ; XIA, D.** *Geometric Calibration of a Micro-CT Systemand Performance for Insect Imaging,* 20. Mai 2016, http://ieeexplore.ieee.org/stamp/stamp.jsp?arnumber=5871720
- **MERY, D. ; GEISERT, C. ; FILBERT, D.** Geometric Calibration of a X-ray Testing System. Technische Universität, 12. Dezember 2012
- **STOPP, F. ; WIECKOWSKI, A. J. ; KÄSEBERG, M. ; ENGEL, S. ; FEHLHABER, F. ; KEEVE, E. A.** Geometric Calibration Method for an Open Cone-Beam CT System. Charit'e Universitäts-medizin, Dezember 2012
- **SUN, Y. ; HOU, Y. ; ZHAO, F. ; HU, J.** A Calibration Method for Misaligned Scanner Geometry in Cone-beam Computed Tomography. Technische Universität, Dezember 2012
- Calibration method of robot base frame using unit quaternion form. **WANG, W. ; LIU, F. ; YUN, C.** Technical report. Central Michigan University
- Base frame calibration for coordinated industrial robots. **YAHUI, G. ; XIANZHONG, D.** Technical report, School of Automation. Southeast University, 2011
- **YANG, K. ; KWAN, A. L. C. ; MILLER, D. F. ; BOONEA, J. M.** *A geometric calibration method for cone beam CT systems,* 20. Mai 2016, http://www.ncbi.nlm.nih.gov/pmc/articles/PMC2840998
- **ZHAO, J. ; HU, X. ; ZOU, J. ; HU, X.** *Geometric Parameters Estimation and Calibration in Cone-Beam Micro-CT,* http://www.mdpi.com/1424-8220/15/9/22811/pdf